(19) Europäisches Patentamt
European Patent Office
Office européen des brevets

(11) **EP 3 854 434 A1**

(12) **EUROPÄISCHE PATENTANMELDUNG**

(43) Veröffentlichungstag:
**28.07.2021 Patentblatt 2021/30**

(51) Int Cl.:
***A61M 5/30*** *(2006.01)*       ***A61M 5/20*** *(2006.01)*
***A61D 1/02*** *(2006.01)*

(21) Anmeldenummer: **20211511.9**

(22) Anmeldetag: **03.12.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **06.12.2019 DE 102019133394**

(71) Anmelder: **UMS GmbH & Co. KG**
**97647 Willmars (DE)**

(72) Erfinder:
• **WANNENWETSCH, Alexander**
**97647 Willmars (DE)**
• **JÜGELT, Lutz**
**98631 Jüchssen (DE)**

(74) Vertreter: **Pöhner, Wilfried Anton**
**Pöhner Scharfenberger & Partner**
**Patent- und Rechtsanwälte mbB**
**Kaiserstrasse 33**
**97070 Würzburg (DE)**

(54) **IMPFPISTOLE MIT FLÜSSIGKEITSSENSOR IN DER DRUCKKAMMER**

(57)    Vorliegende Erfindung betrifft Impfpistole zur Hochdruckinjektion einer Impfflüssigkeit durch die Haut eines Lebewesens, umfassend eine Hochdruckkammer mit einem Einlass umfassend ein Einlassventil, wobei an dem Einlass eine Zuleitung angeschlossen ist, über welche die Impfflüssigkeit während eines Ladevorgangs aus einem Reservoir in die Hochdruckkammer fließen kann, und einem Auslass umfassend ein Auslassventil, wobei während eines Ausstoßvorgangs in der Hochdruckkammer vorhandene und durch ein Druckerzeugungsmittel unter Hochdruck versetzte Impfflüssigkeit durch den Auslass ausfließen und aus der Impfpistole als scharf gebündelter Flüssigkeitsstrahl austreten kann, und einen Flüssigkeitssensor, welcher das Vorhandensein oder Nichtvorhandensein von Flüssigkeit in dem Hochdruckkammerinnenraum und/oder einer Flüssigkeitsströmung durch den Einlass in den Hochdruckkammerinnenraum hinein oder aus diesem heraus während des Ladevorgangs oder des Ausstoßvorgangs misst, und eine Steuerung, welche aus den Ergebnissen der Messung des Flüssigkeitssensors das Vorliegen oder Nichtvorliegen eines Fehlerzustandes ableitet und ein Auslösen des Lade- und/oder Ausstoßvorgangs nur freigibt, sofern kein Fehlerzustand vorliegt, wobei der Flüssigkeitssensor in direkter fluider Kommunikation mit dem Innenraum der Hochdruckkammer steht, wobei der Sensorkanal an einer Stelle einer Innenfläche der Hochdruckkammer mündet, an welcher eine lokale Strömung mit einer Strömung von Impfflüssigkeit durch den Einlass in die Hochdruckkammer hinein oder aus der Hochdruckkammer heraus korreliert ist. Die Erfindung betrifft weiterhin ein Verfahren zur Erkennung eines Fehlerzustandes in einer solchen Impfpistole.

Fig. 1

**Beschreibung**

Vorliegende Erfindung betrifft eine Impfpistole zur Hochdruckinjektion einer Impfflüssigkeit durch die Haut eines Lebewesens, umfassend eine Hochdruckkammer mit einem Einlass umfassend ein Einlassventil, wobei an dem Einlass eine Zuleitung angeschlossen ist, über welche die Impfflüssigkeit während eines Ladevorgangs aus einem Reservoir in die Hochdruckkammer fließen kann, und einem Auslass umfassend ein Auslassventil, wobei während eines Ausstoßvorgangs in der Hochdruckkammer vorhandene und durch ein Druckerzeugungsmittel unter Hochdruck versetzte Impfflüssigkeit durch den Auslass ausfließen und aus der Impfpistole als scharf gebündelter Flüssigkeitsstrahl austreten kann, und einen Flüssigkeitssensor, welcher das Vorhandensein oder Nichtvorhandensein von Flüssigkeit in dem Hochdruckkammerinnenraum und/oder einer Flüssigkeitsströmung durch den Einlass in den Hochdruckkammerinnenraum hinein oder aus diesem heraus während des Ladevorgangs oder des Ausstoßvorgangs misst, und eine Steuerung, welche aus den Ergebnissen der Messung des Flüssigkeitssensors das Vorliegen oder Nichtvorliegen eines Fehlerzustandes ableitet und ein Auslösen des Lade- und/oder Ausstoßvorgangs nur freigibt, sofern kein Fehlerzustand vorliegt. Die Erfindung betrifft weiterhin ein Verfahren zur Erkennung eines Fehlerzustandes in einer solchen Impfpistole.

Zur Injektion von Medikamenten, insbesondere bei Impfungen, werden besonders in der Veterinärmedizin gerne sogenannte Impfpistolen eingesetzt, welche zur Penetration der Haut des zu impfenden Lebewesens keine Nadel, sondern einen scharf gebündelten Flüssigkeitsstrahl einsetzten. Dieser wird dadurch erzeugt, dass die zu injizierende Flüssigkeit in einer Druckkammer der Impfpistole durch ein Druckerzeugungsmittel, üblicherweise ein axial verschieblicher Kolben, aus der Druckkammer herausgepresst wird, wobei die die Druckkammer verlassende Flüssigkeit durch eine Düse an der Mündung der Impfpistole zu einem scharfen Flüssigkeitsstrahl gebündelt wird. Durch Einsatz von ausreichend hohen Drücken von üblicherweise 250 bar oder mehr kann dieser Flüssigkeitsstrahl die Haut auch ohne eine Nadel penetrieren und in das darunter liegende Gewebe gelangen.

Impfpistolen haben den Vorteil, dass aufgrund des Verzichts auf eine Nadel mehrere Impfvorgänge schnell hintereinander ausgeführt werden können, ohne dabei die nötigen Sterilitätsanforderungen zu verletzen. Bei Verwendung einer üblichen Spritze mit Nadel wäre hierbei ein Wechseln der Nadel oder deren Sterilisierung nötig, was deutlich mehr Zeit beansprucht, als das Nachladen der Hochdruckkammer aus einem an die Impfpistole angeschlossenen bzw. einen Teil der Impfpistole bildenden Impfflüssigkeitsreservoir.

Bei Impfpistolen besteht jedoch das Problem, dass deren korrekte Funktion überwacht werden muss. Insbesondere muss sichergestellt sein, dass vor Auslösen des Ausstoßvorgangs die Hochdruckkammer tatsächlich vollständig mit Impfflüssigkeit gefüllt ist. Dies ist insbesondere dann nicht der Fall, wenn das Impfflüssigkeitsreservoir leer ist. Da die bei einem Impfstoß injizierten Volumina recht klein sind, kann die vollständige Entleerung des Reservoirs nicht allein optisch durch den Benutzer kontrolliert werden, etwa über ein transparentes Reservoirgehäuse. Um die üblicherweise teure Impfflüssigkeit möglichst vollständig zu verwenden, sollte vielmehr auch das in den Zuleitungen vom Reservoir zur Hochdruckkammer vorhandene Restvolumen noch vollständig verwendbar sein.

Das Leerlaufen des Reservoirs ist ein regelmäßig zu erwartender Vorgang. Es können jedoch auch unerwartete Fehlerzustände auftreten, die ebenfalls detektiert werden sollten. Beispielsweise ist besonders das nahe der Mündung der Impfpistole im Auslass der Hochdruckkammer positionierte Auslassventil gefährdet, von durch die Mündungsöffnung gelangender Verschmutzung in seiner offenen Stellung blockiert zu werden. In einem solchen Fall würde der Nachladevorgang der Hochdruckkammer in der Weise gestört, dass nicht nur Flüssigkeit aus dem Reservoir durch den Einlass nachströmt, sondern auch Luft durch den fälschlicherweise offenen Auslass in die Hochdruckkammer gelangt, sodass die Hochdruckkammer nach der Beendigung des Aufladevorgangs, beispielsweise dann, wenn der axiale Kolben, der während des Aufladevorgangs als Unterdruckerzeugungsmittel dient, vollständig zurückgezogen ist, nicht nur mit Impfflüssigkeit sondern mit einem Gemisch gefüllt ist, welches neben Impfflüssigkeit mehr oder minder viel Luft umfasst.

Darüber hinaus kann auch das Einlassventil der Hochdruckkammer eine Fehlfunktion aufweisen. Das Einlassventil ist so konstruiert, dass es bei Erzeugen eines Unterdrucks in der Hochdruckkammer ab einer gewissen Druckdifferenz öffnet und so ein Flüssigkeitsstrom aus der Zuleitung in die Hochdruckkammer ermöglicht. Besteht hingegen kein Unterdruck oder gar ein Überdruck in der Hochdruckkammer bleibt das Einlassventil normalerweise geschlossen. Bei einem Defekt kann das Einlassventil jedoch entweder in einer mehr oder minder offenen oder in der geschlossenen Position blockiert sein.

Im ersteren Fall würde bei einem Ausstoßvorgang, wenn das Druckerzeugungsmittel, üblicherweise der Kolben, das im Inneren der Hochdruckkammer befindliche Medium, im Normalzustand zu 100% Impfflüssigkeit, unter einen hohen Druck versetzt, um es durch den Auslass und die Mündung aus der Impfpistole auszustoßen, auch ein Teil des Mediums durch das in offenen Stellung blockierte Einlassventil zurück in die Zuleitung und möglicherweise bis in das Reservoir fließt. Dadurch wird die tatsächlich injizierte Flüssigkeitsmenge unvorteilhaft reduziert, sodass die Injektion einer zur Herstellung eines ausreichenden Impfschutzes nötige Flüssigkeitsmenge nicht garantiert ist.

Ist das Einlassventil hingegen in der geschlossenen Stellung blockiert, so fließt während des Ladevorgangs der Hochdruckkammer keine Flüssigkeit nach und die Hochdruckkammer bleibt somit leer, bzw. lediglich mit durch ein etwaig in der Offenstellung blockiertes Auslassventil eingeströmter Luft gefüllt. In jedem Fall darf bei diesem Fehlerzustand ein Ausstoßvorgang nicht ausgelöst werden, da sonst die Gefahr besteht, dass fälschlicherweise Luft injiziert wird, was nicht nur keine positive Impfwirkung zur Folge hätte, sondern sogar, bei einer ausreichend großen Luftmenge, welche in den Blutkreislauf des zu impfenden Lebewesens gelangt, gesundheitsgefährdend sein könnte.

Um die korrekte Funktion der Impfpistole zu überwachen, ist es im Stand der Technik bekannt, Flüssigkeitssensoren einzusetzen. So offenbart beispielsweise die europäische Patentschrift EP 1515763 B1 eine Impfpistole, bei welcher ein Flüssigkeitssensor in der Zuleitung zur Hochdruckkammer das korrekte Einströmen von Medikamentenflüssigkeit überwacht und eine Betätigung oder Auslösen der Impfpistole nur dann freigegeben wird, sofern während des Ladens der Hochdruckkammer detektiert wurde, dass eine ausreichende Flüssigkeitsmenge aus dem Reservoir durch die Zuleitung in die Hochdruckkammer eingeströmt ist.

Der Vorteil der Positionierung des Flüssigkeitssensors in der Zuleitung besteht darin, dass der hierdurch in einem Bereich der Impfpistole platziert werden kann, in dem mehr Raum für den Sensor sowie die ihn überwachende und steuernde Auswerteelektronik vorhanden ist. Dies ist bei der in der EP 1515763 B1 vorgeschlagenen Realisierung des Flüssigkeitssensor als eine Kombination aus LED und Photodiode von Bedeutung, da ein solcher Sensor schlecht miniaturisierbar ist.

Zudem muss ein solcherart angeordneter Sensor nicht hochdruckstabil sein, da er sich in Nachströmungsrichtung der Flüssigkeit vor dem Einlassventil befindet und von diesem vor dem während des Ausstoßvorgangs in der Hochdruckkammer erzeugten hohen Druck geschützt wird.

Ein wesentlicher Nachteil ist jedoch, dass nicht alle oben beschriebenen Fehlerzustände gleichermaßen deutlich voneinander unterscheidbar sind, da lediglich die Strömung der Flüssigkeit in der Zuleitung gemessen wird, und daraus nur indirekt Informationen über den Zustand in der Hochdruckkammer ableitbar sind.

Vorliegende Erfindung hat sich vor diesem Hintergrund die Aufgabe gestellt, eine Impfpistole mit einem überwachenden Flüssigkeitssensor zu entwickeln, bei der alle möglichen auftretenden Fehlerzustände klar voneinander unterschieden werden können und welche darüber hinaus auch eine vollständigere Ausnutzung der verwendeten Impfflüssigkeitsmenge erlaubt.

Gelöst wird diese Aufgabe durch eine Impfpistole gemäß einem der Ansprüche 1-15, deren Funktion mit dem Verfahren gemäß einem der Ansprüche 16-18 überwacht wird.

Die wesentliche Neuerung gegenüber bekannten flüssigkeitssensorüberwachten Impfpistolen besteht hierbei darin, den Sensor nicht in der Zuleitung zu positionieren, sondern in der Hochdruckkammer bzw. derart, dass er zu jederzeit, d.h. während jeder Phase des Ladens oder Ausstoßes der Impfpistole, in direkter fluider Kommunikation mit der Hochdruckkammer steht. Er ist also insbesondere nicht in der Zuleitung zwischen Einlassventil und Reservoir angeordnet, sondern in Strömungsrichtung vom Reservoir in die Hochdruckkammer gesehen hinter dem Einlassventil. Er ist weiterhin erfindungsgemäß an einer Stelle der Hochdruckkammer positioniert, an welcher die lokale Flüssigkeitsströmung, in Betrag und Richtung, mit der Strömung durch den Einlass, also in die Hochdruckkammer hinein oder, bei einem Defekt des Einlassventils, auch aus dieser heraus, korreliert ist, so dass aus dem erfassten Sensorsignal verlässlich auf die Strömungsverhältnisse am Einlass geschlossen werden kann. Die Korrelation kann hierbei also positiv oder negativ sein, sollte nur betragsmäßig möglichst hoch, d.h. nahe 1 liegen.

Das Einlassventil sitzt üblicherweise in einem Zugang der Hochdruckkammer in Form eines zylinderförmigen Stutzens einer stirnseitigen Öffnung zur Hochdruckkammer hin, wobei die Öffnung einen kurzen Kanal in Form eines Seitenarms der Hochdruckkammer, nachfolgend Einlassöffnung oder -kanal genannt, bildet. Es wird von vorliegender Erfindung insbesondere empfohlen, den Flüssigkeitssensor in einem zusätzlichen, weiteren Zugang zu positionieren, der relativ zu dem Zugang des Einlasses um zwischen 30° und 180°, bevorzugt zwischen 45° und 90°, versetzt an der üblicherweise zylindrisch geformten Hochdruckkammer angebracht ist und eine gleichartige Zugangsöffnung bzw. einen Zugangskanal, im Folgenden Sensoröffnung oder -kanal genannt, aufweist. Der den Flüssigkeitssensor beherbergende weitere Zugang kann insbesondere axial auf gleicher Höhe zum Auslass positioniert sein. Weiterhin können die Mündungen der die Zugänge verbindenden kurzen Kanäle, im Folgenden für den Einlass der Einlasskanal und für den Zugang des Sensors der Sensorkanal genannt, über eine die Hochdruckkammer umlaufende Nut oder Kehlung miteinander verbunden sein. Diese Positionierung des Flüssigkeitssensors und/oder Gestaltung der Hochdruckkammer ermöglicht zum einen eine Messung des Vorhandenseins oder Nichtvorhandenseins von Flüssigkeit in der Hochdruckkammer selbst, zum anderen erhöht sie (betragsmäßig) die Korrelation zwischen lokaler Strömung am Ort des Sensors und der Strömung durch den Einlass.

Als Flüssigkeitssensor wird von vorliegender Erfindung insbesondere ein Sensor vorgeschlagen, welcher zwei Thermistoren, d.h. also zwei mit einem reproduzierbaren temperaturabhängigen Widerstand aufweisende Elemente umfasst. Von diesen zwei Thermistoren wird einer als Referenz und einer als Messthermistor betrieben. Hierbei wird der Messthermistor mittels einer Heizung aufgeheizt, sodass sich eine Temperaturdifferenz zum Referenzthermistor einstellt, welche abhängig ist von den Temperaturleiteigenschaften des Umgebungsthermistors.

Beide Thermistoren werden in dem Flüssigkeitssensor so positioniert, dass sie in direktem thermischen Kontakt mit dem in der Hochdruckkammer vorhandenen Medium stehen. Im Normalzustand ist dies immer Flüssigkeit. Während bei Auftreten eines Fehlerzustandes kann jedoch auch Luft oder ein Vakuum in der Hochdruckkammer vorliegen, was der Flüssigkeitssensor durch ein starkes Ansteigen der Temperaturdifferenz zwischen den beiden Thermistoren bei einer gegebenen Heizleistung anzeigt. Die Strömungsverhältnisse am Ort des Thermistors können ebenfalls aus der Temperaturdifferenz abgelesen werden. Sofern das Medium im Bereich des Flüssigkeitssensors keine Strömung aufweist, stellt sich eine höhere Temperaturdifferenz ein als wenn eine Strömung vorhanden ist, da dem strömenden Medium auch die vom beheizten Thermistor abgegebene Wärme schneller abtransportiert wird und somit also eine Kühlung stattfindet.

Das erfindungsgemäß wird sowohl während des Lade- als auch des Ausstoßvorgangs ein Fehlerzustandserkennungsverfahren durchgeführt.

Während des Ladevorgangs, welcher zumindest eine Einströmphase umfasst, wird mittels des Flüssigkeitssensors in der Hochdruckkammer ein zu einer Ist-Einströmrate proportionales und/oder von der Art des in der Hochdruckkammer vorliegenden Mediums abhängiges Signal gemessen und von der Steuerung der Impfpistole ausgewertet.

Nachdem im Normalbetrieb das Einlassventil geöffnet haben sollte, wird von der Steuerung der Aufbau einer Strömung in die Hochdruckkammer mit einer gewissen Soll-Strömungsrate erwartet. Sofern aus dem Messsignal des Flüssigkeitssensors hervorgeht, dass die Ist-Strömungsrate durch den Einlass sehr viel geringer ist als die Soll-Strömungsrate und/oder sich in der Hochdruckkammer ein Vakuum aufbaut, wird der Fehlerzustand "Reservoir leer ODER Einlassventil defekt geschlossen" erkannt. Bei dem bevorzugten Thermistor-basierten Flüssigkeitssensor ist dies an einer höher bis sehr viel höher als erwarteten Temperaturdifferenz erkennbar. Sofern die Ist-Einströmrate niedriger, jedoch nicht sehr viel niedriger ist als die Soll-Einströmrate und/oder das Vorhandensein von Luft in der Hochdruckkammer angezeigt wird, wird hingegen der Fehler "Auslassventil offen" erkannt. Ein Thermistor-basiert Flüssigkeitssensor zeigt dies durch eine leicht oder deutlich erhöhte Temperaturdifferenz an.

Sobald die Steuerung mit hinreichender Konfidenz das zumindest teilweise Vorhandensein von Luft in der Hochdruckkammer festgestellt hat, blockiert die Steuerung eine Auslösung eines an den Ladevorgang anschließenden Ausstoßvorgangs durch den Benutzer.

Während des Ausstoßvorgangs wird erfindungsgemäß ebenfalls das Messsignal des Flüssigkeitssensors überwacht. Sollte dieses auf eine von Null verschiedene Strömungsrate durch den Einlass hinweisen, welche in diesem Fall aufgrund des hohen Druckunterschieds nur aus der Hochdruckkammer in die Zuleitung gerichtet sein kann, so wird der Fehlerzustand "Einlassventil defekt offen" erkannt.

Der erkannte Fehlerzustand wird dem Benutzer der Impfpistole über Anzeigemittel optisch und/oder akustisch mitgeteilt.

In manchen Ausführungsformen der erfindungsgemäßen Impfpistole kann sich der Ladevorgang in zwei zeitlich klar trennbare Phasen, eine anfängliche Unterdruckerzeugungsphase bis das Einlassventil öffnet anschließend die vorbeschriebene Einströmphase unterteilen.

Bei diesen Ausführungsformen erfolgt die Fehlerzustandserkennung während der Einströmphase des Ladevorgangs sowie während des Ausstoßvorgangs wie oben beschrieben. Zusätzlich erfolgt jedoch auch während der Unterdruckerzeugungsphase eine Überwachung und Auswertung des Flüssigkeitssensorsignals. Wird während der Unterdruckerzeugungsphase bereits eine von Null verschiedene Ist-Strömung registriert, wird der Fehlerzustand "Einströmventil defekt offen" erkannt. Bei einem Flüssigkeitssensor der vorbeschriebenen bevorzugten Art äußert sich dies in einer Temperaturdifferenz welche niedriger ist als erwartet. Wird hingegen festgestellt, dass sich während der Unterdruckerzeugungsphase ein Flüssigkeits-Luft Gemisch bildet, bei einem Thermistor-basierten Flüssigkeitssensor erkennbar anhand einer ansteigenden und den für reine Flüssigkeit zu erwartenden Wert übersteigenden Temperaturdifferenz, wird von der Steuerung bereits in dieser Phase der Fehlerzustand "Auslassventil offen" erkannt. Dieser wird noch bestätigt, also ein in manchen Ausführungsformen von der Steuerung berechneter Konfidenzwert erhöht, wenn in der anschließenden Einströmphase die gemessene Ist-Einströmrate niedriger ist als die Soll-Einströmrate und/oder weiterhin das zumindest teilweise Vorhandensein von Luft registriert wird.

Die Erfindung bietet den Vorteil die eingangs beschriebenen Fehlerzustände besser unterscheiden zu können. Insbesondere ist der Flüssigkeitssensor aufgrund seiner Platzierung in der Hochdruckkammer in der Lage, direkt das gefährliche Vorliegen eines Flüssigkeits-Luft Gemisches in der Hochdruckkammer zu detektieren. Bei dem bevorzugten Flüssigkeitssensor in Form zweier gemeinsam überwachter Thermistoren führt ein solches Gemisch, oder noch mehr reine Luft bzw. Unterdruck, zu einem besonders starken direkten Messsignal. Dahingegen kann bei den im Stand der Technik bekannten Impfpistolen lediglich indirekt auf diesen Zustand geschlossen werden.

Vorteilhafte Weiterbildung vorliegender Erfindung, welche einzeln oder in Kombination realisierbar sind, sofern sie sich nicht gegenseitig offensichtlich ausschließen, sollen nachfolgend kurz vorgestellt werden.

Um trotz der beim Ausstoß auftretenden Druckspitzen von 250 bar oder mehr einen flüssigkeitsdichten Verschluss zwischen der Wandung des Sensors und dem Zugang, in die er eingesetzt ist, zu gewährleisten, wird empfohlen den Sensor in den Zugang zu stutzen, einzupressen oder noch besser einzuschrauben oder mittels eines Bajonettverschlusses zu befestigen. Zusätzlich können auch Dichtmittel wie ein stirnseitiger Dichtring vorhanden sein.

Der Einlass der Hochdruckkammer stellt einen Zugang zu selbiger dar, in welchem zum dichten Verschließen des Einlasses gegen Flüssigkeitsströmungen aus der Hochdruckkammer heraus in die im Einlass angeschlossene Zuleitung und zurück in das Flüssigkeitsreservoir, ein Einlassventil aufweist, welches nur für Flüssigkeitsströmungen aus dem Reservoir in die Hochdruckkammer passierbar ist, und hierbei ab einer Druckdifferenz zwischen Zuleitung und Hochdruckkammer öffnet.

Der Flüssigkeitssensor der erfindungsgemäßen Impfpistole ist bevorzugt in einem weiteren Zugang neben dem Einlass angeordnet. Üblicherweise ist der Einlass in einer dem zylinderförmigen Abschnitt der Innenfläche der Hochdruckkammer positioniert. Dabei kann er radial orientiert sein, oder die Achse, welche durch den Zugang definiert wird, nimmt einen spitzen Winkel mit der Zylinderachse des zylinderförmigen Abschnitts der Hochdruckkammer ein.

Insbesondere kann der Zugang sowohl des Einlasses, als auch der Zugang, in dem der erfindungsgemäße Flüssigkeitssensor angeordnet ist, die Form eines hohlzylinderförmigen oder konischen bzw kegelstumpfartigen Stutzens aufweisen. Im Falle des Einlasses, ist innerhalb des Stutzens das Einlassventil eingebracht. Im Falle des erfindungsgemäßen Flüssigkeitssensors ist dieser in den Stutzen hochdruckflüssigkeitsdicht eingesetzt. Dies kann entweder durch Einpressen, bevorzugt jedoch durch Einschrauben in ein Innengewinde des Zugangsstutzens erfolgen. Auch ein bajonettartiger Verschluss ist denkbar. Um auch bei den auftretenden hohen Drücken von 250 bar oder mehr Dichtheit zu gewährleisten, sind Abdichtungsmittel wie ein oder mehrere Dichtringe, beispielsweise an der Hochdruckkammer zugewandten Stirnseite des Flüssigkeitssensors, empfohlen.

Die Zugänge für den Einlass und den erfindungsgemäßen Flüssigkeitssensor können in dem zylinderförmigen Abschnitt der Hochdruckkammer axial auf gleicher Höhe jedoch tangential um einen Winkel zwischen 30° und 180° zueinander versetzt angeordnet sein. Bevorzugt ist der Zugang des Flüssigkeitssensors um ca. 90° versetzt.

Die Öffnung des Flüssigkeitssensorzugangs in der Innenwand der Hochdruckkammer bildet einen kurzen, von dem Hauptteil der Hochdruckkammer abzweigenden Sensorkanal. Es ist zur möglichst unmittelbaren Messung der Strömung in der Hochdruckkammer sinnvoll, diesen Kanal so kurz wie möglich zu halten. Es wird daher empfohlen, den Winkel zwischen dem Sensorkanal, für welchen ein kreisförmiger Querschnitt vorgeschlagen wird, und der Zylinderachse des zylinderförmigen Abschnitts der Hochdrucckammer möglichst nah an 90° zu wählen. Aufgrund des beschränkten Platzes im Bereich um die Hochdruckkammer der Impfpistole herum ist jedoch eine Orientierung des Sensorkanals von 90° nur schwer realisierbar. Es wird jedoch empfohlen, den Winkel zwischen Sensorkanalachse und Zylinderachse auf jeden Fall nicht kleiner als 45° zu wählen.

Dem Sensorkanal des Flüssigkeitssensorzugangs entspricht der Einlasskanal des Einlasszuganges. Es wird im Rahmen vorliegender Erfindung empfohlen, dass die Mündung dieser beiden Kanäle in dem zylinderförmigen Bereich der Hochdruckkammern über eine Umfangsnut miteinander verbunden sind. Die Tiefe dieser Nut sollte zwischen einem und 10% des Durchmessers des zylinderförmigen Bereichs der Hochdruckkammer betragen, bei üblichen Durchmessern entspricht dies einer Tiefe von 0,2 - 2mm. Durch die Umfangsnut wird vorteilhaft die Strömung aus dem Einlasskanal heraus oder in diesen hinein in Richtung des Sensorkanals geleitet, und so dort besser erfasst und messbar.

Die Umfangsnut kann hierbei eine Vertiefung in der Innenfläche der Hochdruckkammer sein. In manchen Ausführungsformen der erfindungsgemäßen Impfpistole, welche einen im Wesentlichen hohlzylindrischen Einsatz in der Hochdruckkammer vorsehen, um deren effektiven Innendurchmesser zu reduzieren, kann die Umfangsnut auch eine umfängliche Vertiefung in der Mantelfläche des Einsatzes sein.

Aufgrund des bereits angesprochenen begrenzten Raumes im Bereich der Hochdruckkammer und in der Nähe der Mündung der Impfpistole ist es unabdingbar, einen Flüssigkeitssensor von möglichst kompakten Abmessungen einzusetzen. Als besonders effektiv miniaturisierbar wird ein Flüssigkeitssensor umfassend zwei Thermistoren empfohlen. Die Thermistoren sind hierbei im Flüssigkeitssensor so angeordnet, dass sie in möglichst unmittelbarem thermischen Kontakt mit dem in der Hochdruckkammer vorhandenen Medium stehen. Dieses Medium ist im Normalbetrieb üblicherweise Impfflüssigkeit, kann bei dem Auftreten von Fehlerzuständen jedoch auch von außen eingesaugte Luft oder gar ein (teilweises) Vakuum sein.

Einer der Thermistoren dient als Referenzthermistor, dessen Aufgabe es ist, einen der tatsächlichen Temperatur des Mediums entsprechenden Vergleichswert zu liefern. Der andere Thermistor dient als Messthermistor und wird beheizt, um relativ zum Referenzthermistor eine Temperaturdifferenz aufzubauen, deren Höhe Aufschluss über das an den Thermistoren anliegende Medium erlaubt. Bei einem Medium, welches einen besseren Wärmeabtransport ermöglicht, wie beispielsweise Impfflüssigkeit, bildet sich bei einer gegebenen Heizleistung lediglich eine geringere Temperaturdifferenz aus, als bei einem Medium mit einer schlechteren Wärmeleitung, beispielsweise Luft oder Vakuum. Zusätzlich hat auch die Strömung am Ort des Flüssigkeitssensors bzw. der Thermistoren, welche die aktiven Elemente des Sensors darstellen, einen Einfluss auf die Temperaturdifferenz zwischen den Thermistoren, wobei in Abwesenheit von Strömung eine höhere Temperaturdifferenz vorliegt als mit Strömung.

Die Thermistoren können hierbei entweder einen positiven oder negativen Temperaturkoeffizienten aufweisen. Bei Thermistoren mit positiven Temperaturkoeffizienten, oder PTC-Thermistoren, erhöht sich der Widerstand mit der Temperatur. Dies ist das übliche Verhalten der meisten Materialien, ein Thermistor hat jedoch, um ein möglichst starkes Messsignal zu liefern, vorteilhaft eine möglichst große Änderung des Widerstandes mit der Temperatur. Als PTC-Thermistor für den Einsatz im Flüssigkeitssensor der erfindungsgemäßen Impfpistole wird beispielsweise Platindraht vorgeschlagen.

Bevorzugt wird jedoch ein Thermistor mit negativen Temperaturkoeffizienten (NTC-Thermistor) eingesetzt, bei dem sich, zumindest in einem bestimmten Temperaturbereich, die Leitfähigkeit mit der Temperatur erhöht, entsprechend also der Widerstand mit der Temperatur vermindert. Bekannte NTC-Thermistoren basieren üblicherweise auf Silizium.

Der Vorteil von NTC-Thermistoren vor PTC-Thermistoren liegt in der besseren Miniaturisierbarkeit. Da sich bei PTC-Thermistoren der Widerstand mit steigender Temperatur erhöht, reduziert sich, bei vorgegebener Spannung, die im Thermistor umgesetzte (Heiz-)Leistung. Bei NTC-Thermistoren ist das Umgekehrte der Fall, d.h. die Heizleistung bei fester Spannung steigt, je wärmer der Thermistor wird. Hierdurch ist das Erreichen einer möglichst hohen Temperaturdifferenz zwischen Referenz und Messthermistor, was im Rahmen vorliegender Erfindung zum Erhalt eines möglichst starken Messsignals gewünscht ist, vorteilhaft erleichtert. Die Heizleistung berechnet sich bei gegebener Spannung nach der

$$\text{Formel } P_{\text{Heiz}} = \frac{U^2}{R}.$$

Die durch die Heizung zwischen den Thermistoren aufgebaute Temperaturdifferenz beträgt bevorzugt mindestens 3 Kelvin, noch mehr bevorzugt mindestens 10 Kelvin.

Im Falle eines PTC-Thermistors wird aufgrund des vorbeschriebenen Effekts der sich mit steigender Temperatur verminderten Heizleistung empfohlen, ein zusätzliches Heizelement vorzusehen, beispielsweise ein Heizdraht in direkter Nähe zum Thermistor. Ein solches Heizelement erhöht die Komplexität des Flüssigkeitssensors und benötigt Bauraum, weswegen Flüssigkeitssensoren mit NTC-Thermistoren hier im Vorteil sind. Diese können ausschließlich mittels des sie durchfließenden Stroms geheizt werden.

Es besteht nun die Möglichkeit, entweder die Spannung oder die Leistung oder den Heizstrom konstant zu halten. Ersteres führt zu einem Ansteigen der Heizleistung mit Erwärmung des Bauelementes, stellt also ein positives Feedback dar, was zu einem instabilen Verhalten führt. Dies ist daher nicht zu empfehlen. Letzteres führt hingegen zu einer sich im Zuge der Erwärmung vermindernden Heizleistung (Heizleistung $P_{\text{Heiz}} = I^2 * R_{\text{Thermistor}}(T)$), und somit zu einer negativen Rückkopplung, und einem stabilen Verhalten, welches die Temperaturerhöhung des Messthermistors auf natürliche Weise begrenzt. In manchen Ausführungsformen vorliegender Erfindung wird daher das Konstanthalten des Messstroms während der Heizung des Messthermistors empfohlen.

Jedoch ist eine möglichst hohe Temperaturdifferenz zwischen Mess- und Referenzthermistor wünschenswert. In bevorzugten Ausführungsformen vorliegender Erfindung wird daher empfohlen, die Heizleistung $P_{\text{Heiz}}$ bzw $P_{\text{H}}$ während des Heizvorgangs konstant zu halten, und hierbei also die über dem Messthermistor abfallende Spannung mit zunehmender Erwärmung des Thermistors zu senken, jedoch den Strom zu erhöhen gemäß der Abhängigkeiten

$$U = \sqrt{P_{Heiz}} \; x \; r \, (T) \; \text{ bzw. } \; I = \frac{\sqrt{P_{Heiz}}}{r \, (T)}$$

Die Heizleistung gilt im Rahmen vorliegender Erfindung bereits als konstant, wenn sie um weniger als 30% relativ zu einem arithmetischen oder geometrischen Mittel variiert. In bevorzugten Ausführungsformen wird der Messthermistor beispielsweise mit einer konstanten Heizleistung von zwischen 10mW und 20mW betrieben. Je geringer die Abweichungen von der mittleren Heizleistung sind, desto genauer zeigt das ermittelte digitale Ausgabesignal in Form der Temperaturdifferenz pro Heizleistung die Strömungsverhältnisse am Ort des Flüssigkeitssensors an

In manchen Ausführungsformen wird NTC-Messthermistor verwendet, der bei 10°C einen elektrischen Widerstand von, ca. 18 kΩ und bei 30°C einen Widerstand von lediglich ca. 8 kΩ aufweist

Die Thermistoren sind über eine analoge Ansteuer- und Signalerfassungsschaltung an die Steuerung der Impfpistole angeschlossen. Als analoges Messsignal kann, etwa mittels einer Brückenschaltung, ein zur Temperaturdifferenz der Messthermistoren proportionale Spannungssignal erfasst werden. Alternativ kann auch für beide Thermistoren getrennt die über ihnen abfallende Spannung erfasst werden. Die Steuerung digitalisiert das/die analogen Signale und wertet sie mittels eines Steuerungsalgorithmus aus.

Die Heizung des Messthermistors sowie die Erfassung des/r (analogen) Messsignals/e, welches in der Differenz zwischen den über dem Referenzthermistor und dem Messthermistor abfallenden Spannung besteht, können kontinuierlich erfolgen. D.h. der Thermistor wird ohne Unterbrechung beheizt und das Messsignal durchgehend aufgenommen. In der Praxis ist es jedoch so, dass das Messsignal lediglich zu diskreten Zeitpunkten von der digitalen Steuerelektronik der Steuerung, welche den Flüssigkeitssensor ansteuert und überwacht, ausgelesen wird. Um eine weitere Erhöhung der Genauigkeit der Messung zu erreichen, wird daher empfohlen, da die Heizung des Messthermistors vor Erfassung des Messsignals für eine gewisse Zeitdauer zu unterbrechen, um es der im Thermistor erzeugten Wärme zu erlauben, sich gleichmäßig durch den Thermistor und eine ihn einschließende Umhüllung auszubreiten. Hierdurch wird der auf das Medium zurückzuführende Teil der gemessenen Temperaturdifferenz gegenüber dem auf den Effekt einer thermisch isolierenden Umhüllung zurückzuführenden Teil erhöht.

Die Erfassung des Messsignals erfolgt erfindungsgemäß mindestens einmal pro Sekunde, bevorzugt jedoch 10-mal pro Sekunde oder häufiger. Bevorzugt erfolgt die Erfassung periodisch alle 100 ms oder in noch kürzeren Zyklen. Es wird in bevorzugten Ausführungsformen der Erfindung vorgeschlagen, die Heizung für etwa die Hälfte bis zu 80% jedes Zyklus zu aktivieren, und dann auszuschalten, um den vorbeschriebenen Temperaturausgleich in der Umgebung des Thermistors zu ermöglichen. Diese Abschaltzeit sollte zwischen 15% und 30% der Messperiode betragen. Für die Erfassung des Messsignals wird dann für den Rest der Periode, nämlich zwischen 1% - 10% der Periodendauer der Heizstrom bzw. die Heizleistung wieder aktiviert und die sich einstellende über dem Mess-Thermistor abfallende Spannung oder die Differenz dieser Spannung zur der über dem Referenz-Thermistor abfallenden Spannung, als Messsignal erfasst.

Der Referenzthermistor sollte mit einer zeitlich konstantem, im Vergleich zum Mess-Thermistor sehr viel geringeren Leistung beaufschlagt werden. Das Verhältnis der Leistungen von Mess- zu Referenzthermistor beträgt bevorzugt 5:1 oder mehr, besonders bevorzugt 10:1 oder mehr.

Um Sie vor etwaigen chemisch aggressiven Stoffen in der Impfflüssigkeit zu schützen, wird vorgeschlagen, die Thermistoren nicht in unmittelbaren Kontakt mit dem Medium in der Hochdruckkammer, insbesondere der Impfflüssigkeit, treten zu lassen, sondern sie in eine schützende Glashülle einzukapseln. Insbesondere wird vorgeschlagen, die Thermistoren in eine kugelförmige Glashülle einzubetten. Diese sollte, um einen möglichst direkten Thermischen Kontakt zu ermöglichen, so dünn wie möglich gestaltet sein.

Die Abmessungen der erfindungsgemäßen Thermistoren sollten höchstens 1 mm, bevorzugt jedoch 0,5 mm oder weniger, besonders bevorzugt 0,1 mm oder weniger betragen. Entsprechend sollte die sie umhüllende Glaskugel einen Durchmesser von etwas über 1mm, bevorzugt etwas über 0,5mm, besonders bevorzugt etwas über 0,1mm aufweisen. Die die Thermistoren bedeckende Glasschicht sollte an jeder Stelle eine Stärke von mindestens 1 μm aufweisen.

Der Flüssigkeitssensor umfasst bevorzugt ein hohlzylindrisches Rohr, welches mit einer chemisch inerten und mechanisch stabilen Füllmasse ausgefüllt ist, in welche die eingekapselten Thermistoren an einer dem Inneren der Hochdruckkammer zugewandten Stirnseite eingebettet sind. Die Zuleitung zu den Thermistoren sind galvanisch isoliert durch die Verfüllmasse bis zur gegenüberliegenden Stirnseite der Verfüllmasse und dort aus dem Flüssigkeitssensor herausgeführt. Die Zuleitungen sind bevorzugt aus Kupfer oder Silber und haben einen Durchmesser von weniger als 0,2 mm, bevorzugt weniger als 100 μm, besonders bevorzugt weniger als 50 μm.

Das hohlzylindrische Rohr des Flüssigkeitssensors hat bevorzugt eine Länge von weniger als 20 mm, insbesondere weniger als 10 mm, besonders bevorzugt zwischen 1 mm und 6 mm. Der Außendurchmesser des hohlzylindrischen Rohres beträgt bevorzugt 5 mm oder weniger, insbesondere 2 mm oder weniger, besonders bevorzugt zwischen 0,5 und 1mm. Der Innendurchmesser beträgt bevorzugt zwischen 70 und 90 % des Außendurchmessers.

Ein besonderes Problem bei der korrekten Erfassung der Strömung in einer in der Hochdruckkammer vorhandenen Impfflüssigkeit stellt sich dadurch, dass diese wasserbasiert oder ölbasiert sein können. Diese beiden Arten von Impfflüssigkeiten unterscheiden sich deutlich in ihrer Viskosität und damit in den Strömungseigenschaften, wobei ölbasierte Flüssigkeiten deutlich viskoser sind und damit träger reagieren als wasserbasierte. Insbesondere bildet sich bei einer ölbasierten Flüssigkeit nur eine deutlich schwächere Strömung im Sensorkanal aus.

Eine Möglichkeit, dennoch ein ausreichend deutliches Sensorsignal zu erhalten besteht wie vorbeschriebenen darin, den Sensorkanal möglichst kurz auszuführen. Hierzu ist es hilfreich, den Winkel zwischen der Symmetrieachse des Zugangsstutzens, in welchen der Flüssigkeitssensor eingesetzt ist, mit der Zylinderachse der Hochdruckkammer möglichst nahe bei 90° zu wählen. Sofern dies nicht möglich ist, oder um auch im Fall eines rechtwinklig abzweigenden Sensorstutzens den Sensor noch direkter in der Strömung in der Hochdruckkammer zu positionieren, wird vorliegend vorgeschlagen, den Flüssigkeitssensor über seinen Sitz im Sensorstutzen hinaus in den Sensorkanal hineinragen zu lassen.

Je nach Ausführung der Hochdruckkammer ist hierbei aber zu vermeiden, dass der Sensor in den Weg des als Druckerzeugungsmittel dienenden Kolbens hineinragt. Bei Ausführungsformen, mit einem Einsatz mit einer zentralen Bohrung für die Führung des Kolbens, sorgt dieser Einsatz bereits für eine Separation zwischen Kolben und Sensor. Der Sensor, bzw. die aktiven, messwertaufnehmenden Elemente des Sensors kann/können hier also bis nahezu in Kontakt mit dem Einsatz treten und dabei auch über die (zylindrische) Innenwandung der Hochdruckkammer hinausragen, etwa bis in eine den Einsatz umlaufende Umfangsnut hinein. Hier wäre eine besonders stark mit der Strömung durch den Einlass korrelierte Strömung und damit ein sehr deutliches Strömungssignal zu erwarten.

Sofern der Kolben aber bis an besagte zylindrische Innenwandung der Hochdruckkammer reicht, bzw. direkt an dieser entlangfährt, darf der Sensor nicht über diese hervorragen. In den Ausführungsformen der Erfindung, in denen der Sensorstutzen mit der Zylinderachse der Hochdruckkammer aus Raumgründen einen spitzen Winkel einnimmt wird daher vorgeschlagen, den oben beschriebenen zylindrischen Flüssigkeitssensor an seiner in der Hochdruckkammer befindlichen Stirnseite in einem Winkel abzuschrägen, der dem Winkel zwischen Sensorstutzen und Hochdruckkammerachse entspricht, und gerade soweit einzusetzen, dass diese Stirnseite mit der Hochdruckkammerinnenwandung bündig ist.

Die Steuerung des Flüssigkeitssensors sowie der weiteren Funktion der erfindungsgemäßen Impfpistole umfasst eine Steuerungselektronik, welche bevorzugt zumindest einen, in manchen Ausführungen auch zwei oder mehr, Mikrocontroller zur digitalen Verarbeitung der erfassten Messdaten des Flüssigkeitssensors, sowie der Ausführung eines Steuerungsalgorithmus dient. Die die Anweisungen des Steuerungsalgorithmus und hierbei in einem durch den Mikrocontroller abrufbaren Datenspeicher der Steuerelektronik, insbesondere einem EEPROM oder SSD-Speicher abgelegt. Der Datenspeicher kann auch in den Mikrocontroller integriert sein.

In manchen Ausführungsformen der Erfindung ist auch je ein Mikrocontroller zur Ansteuerung des Flüssigkeitssensors und erfassung, inklusivie Digitalisierung des Sensorsignals, und ein weiterer Mikrocontroller zur Steuerung bzw. Überwachung der übrigen Funktionen der Impfpistole, wie beispielsweise dem Druckerzeugungsmittel, dem Auslöser und/oder ggf. den Anzeigemitteln, welche einem Benutzer den Status der Impfpistole und hierbei insbesondere etwaige Fehlerzustände anzeigen.

In Ausführungsformen der erfindungsgemäßen Impfpistole, in denen zwei getrennte Mikrocontroller eingesetzt sind, kann der Algorithmus, der das erfasste und digitalisierte Flüssigkeitssensorsignal mit den Vorgängen der Impfpistole, also Lade- oder Auslösevorgang mit den jeweiligen Phasen, korreliert und hierraus das Vorliegen/Nicht-Vorliegen eines Fehlerzustandes ableitet, entweder auf dem dem Flüssigkeitssensor zugeordneten und diesen ansteuernden Mikrokontroller oder auf dem übergeordneten, die übrigen Funktionen der Impfpistole überwachenden Mikrokontroller ausgeführt werden.

Die Steuerung umfasst weiterhin in einigen Ausführungsformen eine Ansteuer- und Signalerfassungsschaltung, im Folgenden kurz Ansteuerschaltung, über welche die Steuerung den Flüssigkeitssensor mit Strom und Spannung beaufschlagen und ein oder mehrere analoge Messsignale erfassen kann.

In den Ausführungsformen der Erfindung, in denen ein Thermistor-basierter Flüssigkeitssensor eingesetzt wird, kann die Ansteuerschaltung eine Brückenschaltung umfassen, durch welche als analoges Messsignal ein von der Temperaturdifferenz der Thermistoren abhängiges, insbesondere innerhalb eines begrenzten Temperaturbereichs proportionales, Strom- oder Spannungssignal erfassbar ist.

Alternativ kann die Ansteuerschaltung auch für jeden der Thermistoren einen Spannungsteiler vorsehen mittels dessen ein Strom oder bevorzugt Spannungssignal als analoge Messsignale erfassbar sind.

Die mittels der Ansteuerschaltung erfassbaren analogen Messsignale werden zu regelmäßigen, insbesondere periodischen, oder unregelmäßigen Messzeitpunkten von einem Analog-Digital(AD)-Wandler der Steuerung digitalisiert und von einem Mikrokontroller ein digitales Ausgabesignal abgeleitet.

In den bevorzugten Ausführungsformen der Erfindung, in denen ein Thermistor-basierter Flüssigkeitssensor eingesetzt wird, gibt das digitale Ausgabesignal bevorzugt die aktuelle Temperaturdifferenz oder die Temperaturdifferenz geteilt durch die dem Thermistor zugeführte Heizleistung wieder. Die Heizleistung, mit der die Temperaturdifferenz ggf. normiert wird kann hierbei die mittlere Heizleistung während der Heizphase oder der gesamten Messperiode oder die Heizleistung im Messzeitpunkt sein.

Bei dem erfindungsgemäßen Verfahren zur Ermittlung eines Fehlerzustandes in einer erfindungsgemäßen Impfpistole wird das durch Auswertung des mittels des Flüssigkeitssensor ermittelten digitalen Ausgabesignals durch die Steuerelektronik anhand des Steuerungsalgorithmus, welcher auch Anweisungen zur Auswertung des Ausgabesignals umfasst, ausgewertet. Hierbei soll insbesondere abgeleitet werden, welches Medium derzeit an den Sensor anliegt, und somit welches Medium derzeit in der Hochdrucckammer vorhanden ist, und in welchem Strömungszustand sich dieses Medium befindet, insbesondere ob eine Strömung aus dem Einlasskanal in die Hochdruckkammer oder umgekehrt aus der Hochdruckkammer in den Einlasskanal und über das Einlassventil in die Zuleitung zurück vorliegt.

Folgende Tabellen geben den normalen Ablauf sowie das für jeden möglichen (einzelnen) Fehlerzustand erwartete Symptom wieder:

Normaler Ablauf:

| | Vorgang | Sensorsignal |
|---|---|---|
| 1 | Kolben wird zurückgezogen, das Einlassventil ist geöffnet | Flüssigkeit, Strömung |
| 2 | Kolben wird nach vorne gedrückt, Einlassventil geschlossen, Auslassventil geöffnet | Flüssigkeit, keine Strömung |

Mögliche Fehler:

| Fehler | Folge | Sensorsignal |
|---|---|---|
| Auslassventil schließt nicht | es wird Luft über das Auslassventil angesaugt und es fließt kein Impfstoff nach | bei (1) wird keine Strömung erkannt, später wird keine Flüssigkeit detektiert |
| Einlassventil schließt nicht | bei (2) wird Flüssigkeit über den Einlass zurückgedrückt | bei (2) wird eine Strömung erkannt |
| Einlassventil öffnet nicht | es wird keine Flüssigkeit bei (1) angesaugt, es bildet sich ein Vakuum | es wird keine Flüssigkeit detektiert |
| Vorratsbehälter leer | es wird keine Flüssigkeit bei (1) angesaugt | es wird keine Flüssigkeit detektiert |

Im Rahmen der Erfindung ist es nun die Aufgabe der Steuerung, in umgekehrter Richtung, aus dem beobachteten Symptom, also einer Abweichung des zeitlich auf den jeweiligen Operationsvorgang bezogenen Ausgabesignals von seinem erwarteten Verlauf, auf den vorhandenen Fehler zu schließen.

Bei den Ausführungsformen der Erfindung, die einen Thermistor-basierten Flüssigkeitssensor einsetzen, kann hierbei aus einer gemessenen hohen Temperaturdifferenz bzw. die Temperaturdifferenz normiert auf die eingebrachte Heizleistung, mit welcher der Messthermistor beaufschlagt wird, geschlossen werden, dass die Thermistoren und damit der Sensor mit Luft oder Vakuum in Kontakt stehen, und somit keine Impfflüssigkeit in der Hochdruckkammer vorliegt. Eine niedrige Temperaturdifferenz weist jedoch auf das Vorhandensein von Impfflüssigkeit hin. Eine sehr niedrige Temperaturdifferenz zeigt strömende Impfflüssigkeit an. Aus den gemessenen Temperaturdifferenzen und den Eigenschaften der jeweiligen Medien wird durch die Steuerungselektronik eine gemessene Ist-Rate der Strömung sowie ein derzeit vorhandenes Medium abgeleitet. Dies wird während der Operationszyklen der Impfpistole mit den für diese Zyklen erwarteten Soll-Medien bzw. Flüssigkeitsströmungsraten vergleichen und bei Abweichungen, die einen gewissen Toleranzwert übersteigen, einen Fehlerzustand abgeleitet.

Einer der Operationszyklen der Impfpistole ist das Laden der Hochdruckkammer mit Impfflüssigkeit aus dem Flüssigkeitsreservoir. Hierbei wird mittels des Druckerzeugungsmittels der Impfpistole ein Unterdruck in der Hochdruckkammer erzeugt, wodurch Flüssigkeit aus dem Reservoir über die Zuleitung und den Einlass in die Hochdruckkammer gesaugt wird. Das Druckerzeugungsmittel ist üblicherweise ein axial verschieblicher Kolben, welcher das Volumen der Hochdruckkammer entweder vergrößert, nämlich wenn er von der Mündung der Impfpistole weggezogen wird, oder verringert, wenn er auf die Mündung der Impfpistole zu geschoben wird. Der Kolben kann hierbei radiale Bohrungen aufweisen, welche erst ab einer gewissen Axialposition des Kolbens mit radialen Bohrungen in der Hochdruckkammer fluchten, sodass zuvor sich ein Unterdruck im Inneren der Hochdruckkammer aufbaut, der ausreichend groß ist, um, sobald die Radialbohrung des Kolbens fluchten, das Einlassventil schlagartig zu öffnen und während des Einströmens einer ausreichenden Flüssigkeitsmenge offen zu halten. Alternativ kann das Einlassventil sich auch erst bei Überschreiten einer bestimmten Druckdifferenz zwischen Zuleitung und Hochdruckkammer öffnen.

Der Aufladevorgang der Impfpistole unterteilt sich in beiden Fällen in zwei Phasen, nämlich der Unterdruckerzeugungsphase, in der noch keine Flüssigkeit einströmt, aber durch das Zurückziehen des Kolbens langsam ein Unterdruck aufgebaut wird, und eine zweite Phase, in der das Einlassventil geöffnet hat und Flüssigkeit in die Hochdruckkammer einströmt. Die axiale Kolbenposition kann durch die Steuerungselektronik ebenfalls überwacht werden, um bei der Auswertung der Flüssigkeitssensorsignale eine zeitliche Referenz für den Beginn der einzelnen Phasen zu erhalten. Je nach Auslöseschwelle des Einlassventils kann die Unterdruckphase im Vergleich zur sich anschließenden Einströmphase auch sehr kurz sein, etwa so kurz, dass sich die Unterdruckphase nicht klar von der Einströmphase abgrenzen lässt.

Im Normalzustand sollte während der Unterdruckerzeugungsphase noch im Sensorkanal vorhandene Flüssigkeit strömungslos verharren. Das erwartete Messsignal ist also eine niedrige Temperaturdifferenz zwischen den beiden Thermistoren des Flüssigkeitssensors. In der anschließenden Einströmphase sollte hingegen bei korrekter Funktion Impfflüssigkeit aus den Zuleitungen durch den Einlass in die Hochdruckkammer einströmen, was auch zu einer Strömung im Bereich des Flüssigkeitssensors in der Hochdruckkammer führt. Das erwartete Messsignal ist also eine niedrige Temperaturdifferenz zwischen den Thermistoren des Flüssigkeitssensors. Mögliche Fehlerzustände sind zum einen, dass das Reservoir leer ist, also keine Impfflüssigkeit mehr vorhanden ist, dass das Auslassventil in einer offenen Stellung blockiert ist, oder dass das Einlassventil in einer offenen oder geschlossenen Stellung blockiert ist.

Ist die Temperaturdifferenz während der Einströmphase höher als erwartet, entspricht sie insbesondere dem für ein nicht-flüssiges Medium hinweisenden Wert, zeigt dies die Abwesenheit von einströmender Flüssigkeit an. Es kann also darauf geschlossen werden, dass entweder das Reservoir leer oder das Einlassventil im geschlossenen Zustand blockiert ist. Die Steuerelektronik kann über Ausgabemittel der Impfpistole dem Benutzer einen entsprechenden Hinweis geben.

Ist die gemessene Temperaturdifferenz sogar deutlich höher als der für eine stehende Flüssigkeit erwartete Wert, kann darauf geschlossen werden, dass das Auslassventil im offenen Zustand blockiert ist, wodurch sich zum einen bei Zurückziehen des Kolbens kein ausreichender Unterdruck aufgebaut hat, sondern Luft von außen in die Hochdruckkammer eingeströmt ist. Dieser Fehlerzustand führt zu einem sehr deutlichen Signal am Ort des Flüssigkeitssensors, was ein besonderer Vorteil gegenüber den im Stand der Technik bekannten Impfpistolen darstellt. Diese haben, wie eingangs beschrieben, den Flüssigkeitssensor in der Zuleitung, also vor dem Einlassventil, und können lediglich anhand einer etwas verminderten Flüssigkeitsströmung auf den Fehler "Auslassventil offen" schließen. Ob das Einlassventil im offenen Zustand blockiert, nachdem Flüssigkeit eingeströmt ist, kann hingegen zu diesem Zeitpunkt noch nicht festgestellt werden. Dies gilt allerdings auch für die im Stand der Technik bekannten Impfpistolen.

Der "Einlassventil blockiert offen"-Fehler kann erst im Zuge der an das Laden der Hochdruckkammer anschließenden Ausstoßzyklus erkannt werden. Er äußert sich dadurch, dass während des Ausstoßzyklus nicht wie erwartet am Ort des Flüssigkeitssensors ein einer strömungsfreien Flüssigkeit entsprechendes Temperatursignal erfasst wird, sondern die Temperaturdifferenz gegenüber dem erwarteten Wert signifikant erniedrigt ist, was auf eine Strömung aus der Hochdruckkammer durch den Einlass zurück in die Zuleitung hindeutet. Dies ist nur möglich, wenn das Einlassventil im mehr oder minder weit geöffneten Zustand blockiert ist. Auch dieser Zustand ist sehr wichtig zu erkennen, da hierbei die tatsächlich ausgestoßene und im Zuge der Impfung abgegebene Flüssigkeitsmenge niedriger ist als erwartet. Es ist also unter Umständen keine vollständige Impfung erfolgt. Dies ist im Rahmen vorliegender Erfindung durch die Positionierung des Flüssigkeitssensors in der Hochdruckkammer an einer Position, an der die Strömung mit der Strömung durch den Einlass korrigiert ist, gewährleistet.

Weitere Einzelheiten, Vorteile und Merkmale vorliegender Erfindung ergeben sich aus dem im Folgenden anhand der Figuren näher erläuterte Ausführungsbeispiele. Diese sollen die Erfindung lediglich illustrieren und in keiner Weise in ihrer Allgemeinheit einschränken.

Es zeigen:

Figur 1: In zwei Teilfiguren, einen schematischen Längsschnitt durch den und eine Vorderansicht des Mündungsbereichs einer Ausführungsform einer erfindungsgemäßen Impfpistole.

Figur 2: In zwei Teilfiguren, einen Längsschnitt und eine Vorderansicht eines Flüssigkeitssensors mir zwei Thermistoren zur Verwendung in Ausführungsformen der erfindungsgemäßen Impfpistole.

Figur 3A: Eine bevorzugte Ausführungsform einer Ansteuerschaltung für einen Flüssigkeitssensor mit zwei NTC-Thermistoren.

Figur 3B Schematisch den zeitlichen Verlauf der Heizleistung mit der der Messthermistor in bevorzugten Ausführungsformen der Erfindung beaufschlagt wird.

Figur 4: Schematische Darstellung einer Steuerung und der angeschlossenen Komponenten in bevorzugten Ausführungsformen der Erfindung.

Figur 5A: Eine weitere Ausführungsform der Impfpistole bei der die Steuerelektronik im Griff angeordnet ist.

Figur 5B: Ein schematischer Längsschnitt einer Ausführungsform der Impfpistole, bei dem die Steuerungselektronik unmittelbar neben der Hochdruckkammer angeordnet ist.

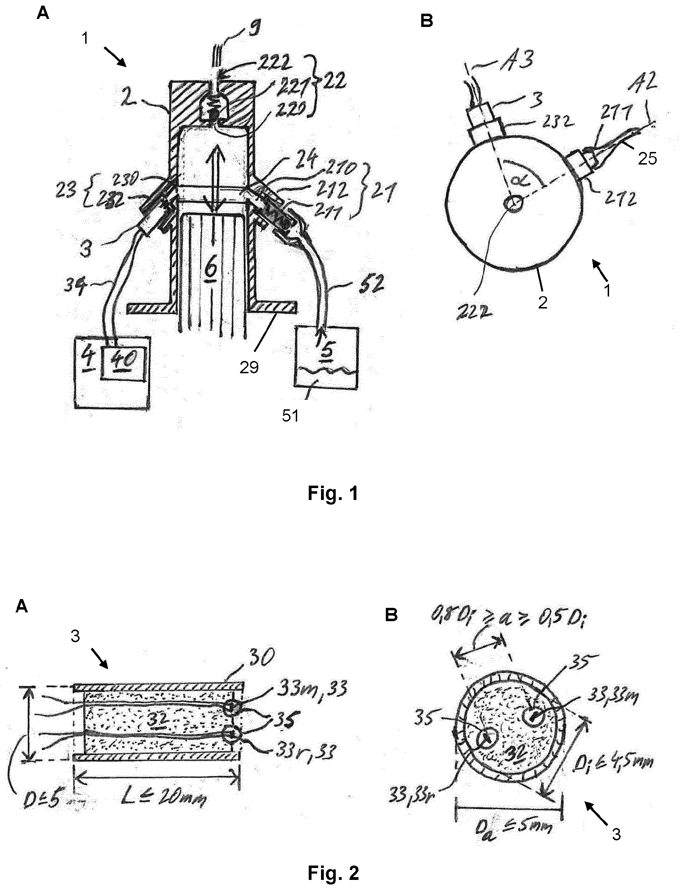

In **Figur 1** ist in zwei Teilfiguren der Mündungsbereich einer bevorzugten Ausführungsform einer erfindungsgemäßen Impfpistole schematisch dargestellt.

**Teilfigur A** zeigt einen schematischen Längsschnitt durch die Hochdruckkammer 2 der Impfpistole 1 sowie ebenfalls schemahaft die über die Verbindungsdrähte 34 an den Flüssigkeitssensor 3 angeschlossene Steuerung 4 sowie das über die Zuleitung 52 an den Einlass 21 angeschlossene Flüssigkeitsreservoir 5, welches Impfflüssigkeit 51 enthält.

**Teilfigur B** zeigt eine Vorderansicht der Impfpistole 1 bzw deren Hochdruckkammer 2, in welcher die zentrale, kreisförmige Mündung 222 des Auslasses 22, sowie zwei seitlich abweisend an der Hochdruckkammer hohlzylindrischen Zugangsstutzen, den Einlassstutzen 212 und den Sensorstutzen 232, mit den darin eingesetzten Komponenten zu erkennen sind. Insbesondere dient Teilfigur B jedoch dazu, den in dieser Ausführungsform vorgesehenen, nahezu rechten, Winkel $\alpha$ zwischen den Symmetrieachsen der Stutzen zu illustrieren, welcher aus Teilfigur A nicht ersichtlich ist, da dort die beiden Stutzen der Einfachheit halber als gegenüberliegend gezeigt sind.

Wie aus der Zusammensicht der beiden Teilfiguren hervorgeht, ist die Hochdruckkammer im Wesentlichen hohlzylindrisch mit einem kreisscheibenförmigen Fuß 26 an einem unteren offenen Ende und einer geschlossenen gegenüberliegenden Stirnseite, welche lediglich eine schmale Öffnung in Form des Auslasses 22 aufweist.

Auslass 22 umfasst die Auslassöffnung 220, das dahinterliegende Auslassventil 221 und anschließende Mündung 222, aus welcher im Hochdruckkammerinneren 20 befindliche Impfflüssigkeit während eines Ausstoßvorgangs, bei welchem sie durch den axial entlang der Zylinderachse der Hochdruckkammer 2 verschiebbaren Kolben 6 unter hohen Druck gesetzt wird, in Form des, hier schematisch angedeuteten, gebündelten Flüssigkeitsstrahls 9 austritt. Im Normalbetrieb kann die Impfflüssigkeit durch den Einlass 21, welcher das in den Einlassstutzen 212 eingesetzte Einlassventil 211 sowie den dahinterliegenden Einlasskanal 210 umfasst, in das Hochdruckkammerinnere 20 fließen. Ist das Einlassventil 211 defekt, kann dieser normale Betrieb gestört sein:

Sofern es im geschlossenen Zustand blockiert ist, kann während des Ladevorgangs der Hochdruckkammer 2 keine Impfflüssigkeit in ihr Inneres 20 nachströmen, wenn der Kolben 6 nach hinten, hier unten, gezogen wird, und es bildet sich im Kammerinneren 20 ein Unterdruck oder es strömt Luft durch ein ebenfalls defektes und im offenen Zustand blockiertes Auslassventil nach. In beiden Fällen erzeugt der erfindungsgemäß über den Sensorkanal 230 zu jeder Zeit in direkter fluider Kommunikation mit dem Kammerinnenraum 20 stehende Flüssigkeitssensor 3 ein deutliches, die zumindest teilweise Abwesenheit von Flüssigkeit anzeigendes analoges Signal, welches von der analogen Ansteuerschaltung 40 der elektronischen Steuerung 4 der Impfpistole 1 erfasst und von der Steuerung 4 digitalisiert und ausgewertet wird. Durch einen Vergleich dieses Ist-Signals mit einem für den jeweiligen Zeitpunkt des Ladezyklus erwarteten Sollwert können die beiden vorgenannten Fehlerzustände "Einlassventil defekt geschlossen" und "Auslassventil offen" sowie der Fehlerzustand "Reservoir leer" erkannt werden.

Ist das Einlassventil im offenen Zustand blockiert, so äußert sich dies während eines Ausstoßvorgangs in Form einer Strömung aus dem Kammerinneren 20 heraus durch den Einlass 21 zurück in die Zuleitung 52. Diese Strömung durch den Einlass 21 ist aufgrund der gewählten relativen Positionen von Einlass 21 und weiterem Zugang 23 mit einer lokalen Strömung im Sensorkanal 230 korreliert, welche durch den Sensor 3 gemessen werden kann. Da in dieser Phase im Normalbetrieb keine Strömung vorliegen sollte, kann die Steuerung somit auf den Fehlerzustand "Einlassventil defekt offen" eindeutig erkennen.

Die Korrelation der Strömungen durch den Einlass (ob in die Kammer 2 hinein oder aus ihr heraus) und im Sensorkanal 230 wird durch die Nut 24, welche eine sich zumindest zwischen dem Sensor und dem Einlasskanal erstreckende Vertiefung in der Innenwandung der Hochdruckkammer 2 darstellt, noch vorteilhaft erhöht.

**Figur 2** zeigt in Teilfigur A einen Längsschnitt und in Teilfigur B eine Vorderansicht eines Thermistor-basierten Flüssigkeitssensors zur Verwendung in bevorzugten Ausführungsformen der erfindungsgemäßen Impfpistole.

Wie hieraus ersichtlich besteht der Flüssigkeitssensor 3 aus einem hohlzylindrischen, bevorzugt metallischen, Rohr 30, welches die zum hochdruckflüssigkeitsdichten einsetzen, bevorzugt Einschrauben in einen Zugangsstutzen der Hochdruckkammer nötige Stabilität verleiht, und welches zum Großteil mit einer Füllmasse 32 ausgefüllt, insbesondere ausgegossen ist. Die Füllmasse 32 ist chemisch inert, insbesondere durch übliche Impfflüssigkeiten nicht angreifbar, schlecht wärmeleitend und bevorzugt auch elektrisch nichtleitend. In einer Stirnseite des zylindrischen Füllmasse-Blocks 32 sind zwei in kugelförmige Glasumhüllungen 35 eingekapselte Thermistoren 33, bevorzugt NTC-Thermistoren, so eingebettet, dass sie zumindest teilweise, hier dargestellt etwa zur Hälfte, aus der Füllmasse 32 herausragen. Grundsätzlich sollten die Glaskugeln 35 so weit hervorragen, wie es noch mit einem sicheren Sitz in der Füllmasse 32 vereinbar ist, um die für den Wärmeaustausch verfügbare Kontaktfläche mit dem im Hochdruckkammerinneren vorhandenen Medium zu maximieren.

Die möglichst platzsparende Ausführung des Sensors 3 ist im Rahmen vorliegender Erfindung von größter Bedeutung, zum einen da der im Mündungsbereich von Impfpistolen vorhanden Raum sehr begrenzt ist, was insbesondere die Länge L des Sensors 3 limitiert. Die Länge sollte daher nicht mehr als 20 mm, bevorzugt deutlich weniger betragen. Aufgrund der Tatsache, dass NTC-Thermistoren mit sehr kleinen Abmessungen herstellbar sind, wäre es grundsätzlich möglich, die Länge auf einige Millimeter, z.B. ca. 2 mm zu begrenzen. Hierbei könnte aber die Handhabung leiden, so dass Länge von um 5 mm bevorzugt ist.

Eine Begrenzung des Durchmessers D, genauer des Außendurchmesser des Rohres 30 ergibt sich aus dem Bestreben, die zur Fixierung des Sensors 3 in seinem Sitz in einem Zugangsstutzen der Hochdruckkammer nötige Kraft zu minimieren. Hierzu soll die dem Druck ausgesetzte Stirnfläche minimiert werden. Es wird daher vorgeschlagen, den Durchmesser D nicht größer als 5 mm, bevorzugt kleiner als 2 mm zu wählen. Der Innendurchmesser Di sollte zwischen 70 - 90 des Außendurchmessers Da betragen und somit nicht größer als 4,5 mm gewählt werden.

Der Abstand a zwischen den Mitten der Kugeln 35 ist bevorzugt zwischen dem 0,5 fachen und dem 0,8 fachen des Innendurchmesser Di des Rohres 30 gewählt, was die Wärmeübertragung vom wärmeren Messthermistor 33m durch die Füllmasse 32 und auch über das recht gut wärmeleitende Rohr 30 auf den kühleren Referenzthermistor 33r minimiert.

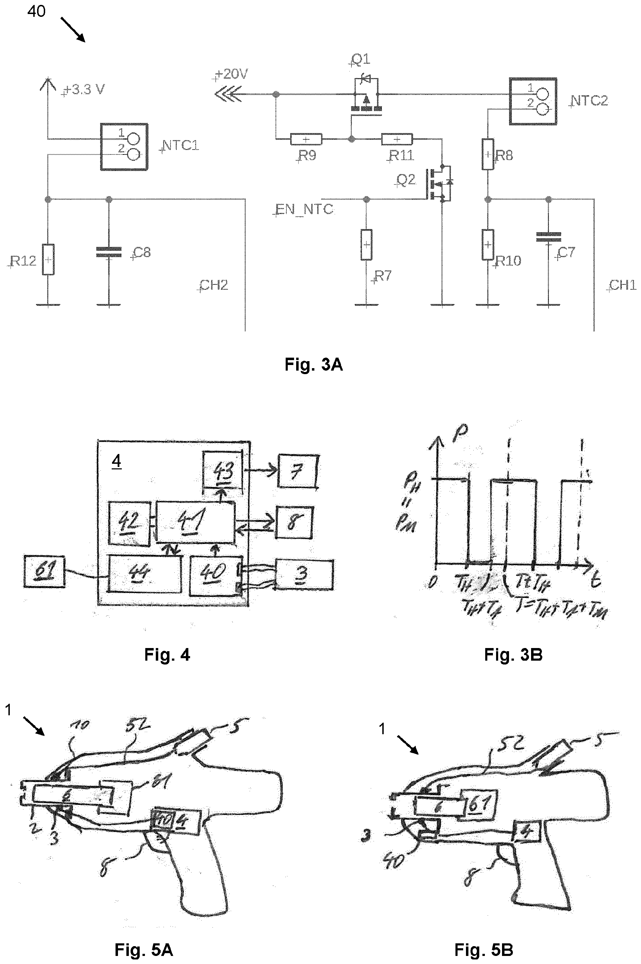

**Figur 3A** zeigt eine bevorzugte Form einer Ansteuerschaltung für einen Thermistor-basierten Flüssigkeitssensor wie den in der Figur 2 dargestellten.

NTC1 ist der Referenz-Thermistor und bildet zusammen mit dem Widerstand R12 einen Spannungsteiler. Das an diesem Spannungsteiler abgegriffene Spannungssignal CH2 ist abhängig von dem sich mit der Temperatur des Mediums ändernden Widerstand von NTC1.

NTC2 bildet ebenfalls einen Spannungsteiler mit den in Reihe geschalteten Widerständen R8 und R10, wird allerdings mit wesentlich höherer Spannung gespeist, damit NTC2 gegenüber dem Referenzthermistor NTC1 möglichst stark erwärmt.

Um den Eingangsspannungsbereich des AD-Wandlers der Steuerung nicht zu überschreiten, erfolgt der Abgriff des Spannungssignals CH1 hier zwischen R8 und R10, die einen weiteren Spannungsteiler darstellen.

R8 und R10 sind so dimensioniert, dass bei unterschiedlichen Temperaturen keine zu großen Unterschiede bei der vom NTC aufgenommenen Leistung auftreten.

Die restliche Schaltung (R7, R9, R11 und Q1 und Q2) dient dazu, den NTC mit einem Steuersignal (EN_NTC) stromlos schalten zu können

Die (analogen) Spannungssignale CH2 und CH1 werden von der (hier nicht dargestellten) Steuerungselektronik erfasst und in ein digitale Signale umgewandelt, welche dann durch den Steuerungsalgorithmus ausgewertet werden können.

In **Figur 3B** ist ein bevorzugter zeitlicher Verlauf der Heizleistung, mit welcher der Messthermistor beaufschlagt wird, graphisch dargestellt.

Auf der Ordinate ist die momentane Leistung P und auf der Abszisse ist die Zeit t abgetragen Während einer ersten Phase einer ersten Periode, welche vom Zeitpunkt $t$=0 bis $t$= $T_H$ andauert, wird der Messthermistor mit einer zeitlich konstanten Heizleistung $P=P_H$ geheizt. Aufgrund der sich im Allgemeinen während dieser Heizdauer ändernden Temperatur, ändert sich auch der elektrische Widerstand des Messthermistors, und somit müssen die Heizspannung sowie der Heizstrom, mit welchem der Thermistor betrieben wird, angepasst werden, um die gewünschte konstante Heizleistung zu erhalten. Diese Anpassung wird durch die Steuerung der Impfpistole vorgenommen.

An die Aufheizphase schließt sich eine Ausgleichsphase von einer Dauer $T_A$ an, während der sich Temperaturgradient, der sich durch die Heizung des Thermistors in der diesen einschließenden Glaskugel vom Thermistor zum Rand hin aufgebaut hat, reduziert, wodurch ein genauerer Temperaturwert erfassbar wird. Die Messwertaufnahme, d. h. Erfassung und Digitalisierung der analogen Messsignale CH1 und CH2, aus welchen die Temperatur erfolgt in der Messphase nach der Ausgleichsphase und dauert lediglich ca. $T_M$=1 ms oder weniger.

In **Figur 4** sind schematisch die Komponenten der Steuerung von bevorzugten Ausführungsformen der erfindungsgemäßen Impfpistole dargestellt.

Die Steuerung 4 umfasst hierbei als zentrales Element den Mikrocontroller 41, der mit dem Datenspeicher 42 in Verbindung steht um Programmbefehle eines Steuerungsalgorithmus zu laden oder errechnete Ergebnisse und Zwischenergebnisse beim Ausführen des Algorithmus abzuspeichern. Von der Ansteuerschaltung 40 erhält der Mikrocontroller die erfassten Messsignale des Flüssigkeitssensors 3. Über Ansteuerschaltung 44 kontrolliert der Mikrocontroller 41 den Kolbenantrieb 61 bzw. erhält von diesem Rückmeldung über die aktuelle Stellung des Kolbens, was als zeitliche Referenz bei der Auswertung der Flüssigkeitssensorsignale dient. Abhängig von den Ergebnissen der Auswertung, also etwa davon, ob ein Fehlerzustand erkannt wurde, wird der Auslöser 9 der Impfpistole blockiert und/oder über die Ansteuerung 43 und Anzeigemittel 7 eine Fehlermitteilung ausgegeben.

Die Figuren 5A und 5B zeigen in schematischen Längsschnitten zwei mögliche Platzierungen der Steuerung 4.

In beiden Teilfiguren sind das Gehäuse 10 der Impfpistole 1 gezeigt, aus dessen linkem Ende die Mündung der Druckkammer 2 freigibt. Der in fluider Kommunikation mit der Druckkammer 2 stehende Flüssigkeitssensor 3 befindet sich innerhalb des Gehäuses 10. In der Kammer 2 befindliche Flüssigkeit kann durch den Kolben 6, der durch den Antrieb 61 bewegt wird, aus der Impfpistole herausgedrückt werden.

In **Figur 5A** ist die Steuerung 4, wie im Stand der Technik bekannt, in der Nähe des Auslösers 8 platziert, wo mehr Raum zur Unterbringung der physischen Komponenten der Steuerung 4 vorhanden ist. In **Figur 5B** ist die Steuerung hingegen in dem Zwischen Außenwandung der Hochdruckkammer 2 und dem Gehäuse 10 angeordnet. Diese von vorliegender Erfindung bevorzugte Positionierung der Steuerung 4 ist bei Verwendung eines Flüssigkeitssensors 3 nach der in Figur 2 gezeigten Art aufgrund der hervorragenden Miniaturisierbarkeit eines solchen Sensors vorteilhaft möglich.

**Bezugszeichenliste**

| | |
|---|---|
| 1 | Impfpistole |
| 2 | Hochdruckkammer |
| 20 | Innenraum von 2 |
| 21 | Einlass |
| 210 | Einlasskanal |
| 211 | Einlassventil |

| 212 | Einlassstutzen |
| 22 | Auslass |
| 220 | Auslassöffnung |
| 221 | Auslassventil |
| 222 | Mündung |
| 23 | weiterer Zugang für 3 |
| 230 | Sensorkanal |
| 232 | Sensorstutzen |
| 3 | Flüssigkeitssensor |
| 30 | Rohr |
| 32 | Füllmaterial |
| 33 | Thermistoren |
| 33r, NTC1 | Referenzthermistor |
| 33m, NTC2 | Messthermistor |
| 35 | kugelförmige Umhüllung von 33 |
| 4 | Steuerung |
| 40 | Ansteuerschaltung für 3 |
| 41 | Mikrocontroller |
| 42 | Datenspeicher |
| 43 | Ansteuerung von 7 |
| 44 | Antriebsansteuerung |
| 5 | Reservoir |
| 51 | Flüssigkeit |
| 52 | Zuleitung |
| 6 | Kolben |
| 61 | Kolbenantrieb |
| 7 | Anzeigemittel |
| 8 | Auslöser |
| 9 | Flüssigkeitsstrahl |
| A2 | Symmetrieachse von 21 |
| A3 | Symmetrieachse von 23 |
| $\alpha$ | Winkel zwischen A2 und A3 |
| D, Da | Außendurchmesser von 30 |
| Di | Innendurchmesser von 30 |
| a | Abstand zwischen |
| R7, R8, R9, R10, R11 | elektrische Widerstände |
| C7, C8 | Kondensatoren |
| Q1, Q2 | elektronische Schalter |
| CH1, CH2 | Spannungssignale |

## Patentansprüche

1. Impfpistole zur Hochdruckinjektion einer Impfflüssigkeit durch die Haut eines Lebewesens, umfassend

   - eine Hochdruckkammer (2) mit

     ◦ einem Einlass (21) umfassend ein in einen Zugangsstutzen (212) eingesetztes Einlassventil (211) und eine mit dem Innenraum (20) der Hochdruckkammer (2) kommunizierenden Einlassöffnung (210), wobei an dem Einlass (21) eine Zuleitung (52) angeschlossen ist, über welche die Impfflüssigkeit (IF) während eines Ladevorgangs aus einem Reservoir (5) in die Hochdruckkammer (2) fließen kann, und

     ◦ einem Auslass (22) umfassend einen Auslasskanal (220), ein Auslassventil (221) und eine Mündung (222), wobei während eines Ausstoßvorgangs in der Hochdruckkammer (2) vorhandene und durch ein Druckerzeugungsmittel, insbesondere einem entlang einer Längsachse der Hochdruckkammer (2) axial verschieblichen Kolben (6), unter Hochdruck versetzte Impfflüssigkeit durch den Auslasskanal (220) und das Auslassventil (221) aus der Hochdruckkammer (2) ausfließen und durch eine hinter dem Auslassventil (221) gelegene Mündung (222) der Impfpistole (1) als scharf gebündelter Flüssigkeitsstrahl (9) austreten kann, und

- einen Flüssigkeitssensor (3), welcher das Vorhandensein oder Nichtvorhandensein von Flüssigkeit in dem Hochdruckkammerinnenraum (20) und/oder einer Flüssigkeitsströmung durch den Einlass (21) in den Hochdruckkammerinnenraum (20) während des Ladevorgangs oder des Ausstoßvorgangs misst, und

- eine Steuerung (4), welche aus den Ergebnissen der Messung des Flüssigkeitssensors (3), das Vorliegen oder Nichtvorliegen eines Fehlerzustandes ableitet und dies einem Benutzer anzeigt und/oder ein Auslösen des Lade- und/oder Ausstoßvorgangs nur freigibt, sofern kein Fehlerzustand vorliegt,

**dadurch gekennzeichnet, dass**
der Flüssigkeitssensor (3) in direkter fluider Kommunikation mit dem Innenraum (20) der Hochdruckkammer (2) steht, wobei der Sensorkanal an einer Stelle einer Innenfläche der Hochdruckkammer (2) mündet, an welcher eine lokale Strömung mit einer Strömung von Impfflüssigkeit durch den Einlass (21) in die Hochdruckkammer (2) hinein oder aus der Hochdruckkammer (2) heraus korreliert ist.

2. Impfpistole nach Anspruch 1, **dadurch gekennzeichnet, dass** der Flüssigkeitssensor in einem weiteren Zugang (23) neben dem Einlass (21) angeordnet ist und über einen Seitenarm der Hochdruckkammer bildenden Sensorkanal (230) mit dem Hochdruckkammerinnenraum (20) verbunden ist.

3. Impfpistole nach Anspruch 2, **dadurch gekennzeichnet, dass** der Einlass (21) und der den Flüssigkeitssensor (3) beherbergende weitere Zugang (23) in einem zylinderförmigen Abschnitt der Innenfläche der Hochdruckkammer (2), insbesondere tangential um ca. 90° versetzt aber axial auf gleicher Höhe, angeordnet sind, wobei der weitere Zugang (23) insbesondere einen nach außen weisenden zylindrischen oder konischen Zugangsstutzen (232) umfasst, der eine Symmetrieachse aufweist, die mit der Zylinderachse des zylindrischen Abschnitts der Hochdruckkammerinnenfläche einen Winkel von zwischen 30 und 90 insbesondere von zwischen 45°-90° einnimmt, wobei der Flüssigkeitssensor (3) mit einem zu einem Innenraum des Stutzens (232) komplementär geformten Ende hochdruckflüssigkeitsdicht in diesen eingesetzt ist und insbesondere in den Sensorkanal (230) hineinragt.

4. Impfpistole nach einem der Ansprüche 1-3, **dadurch gekennzeichnet, dass** der Flüssigkeitssensor (3) zwei Thermistoren (33) umfasst, welche mit einem im Innenraum (20) der Hochdruckkammer (2) vorhandenen Medium, insbesondere Luft oder Impfflüssigkeit oder einem Impfflüssigkeits-Luft-Gemisch, beispielsweise in Form eines Schaumes, in direktem thermischen Kontakt steht.

5. Impfpistole nach Anspruch 4, **dadurch gekennzeichnet, dass** einer der Thermistoren (33) als Referenzthermistor (33r, NTC1) und der andere Thermistor (33) als Messthermistor (33m, NTC2) dient, wobei letzterer beheizt ist, sodass in einem thermischen Gleichgewicht eine Temperatur des Messthermistors (33m, NTC2) um eine gewisse Temperaturdifferenz höher ist als eine Temperatur des Referenzthermistors (33r, NTC1).

6. Impfpistole nach Anspruch 5, **dadurch gekennzeichnet, dass** die Heizung des Messthermistors (33m, NTC2)

- indirekt mittels eines Heizelementes, insbesondere eines elektrisch erwärmten Heizdrahtes, oder
- direkt mittels eines durch den Messthermistor (33m, NTC2) fließenden Stromes, insbesondere eines Messstromes, erfolgt.

7. Impfpistole nach Anspruch 5 oder 6, **dadurch gekennzeichnet, dass** die Temperaturdifferenz in dem Fall, dass das in der Hochdruckkammer (2) vorhandene Medium Luft ist, mindestens 3 Kelvin, insbesondere mindestens 10 Kelvin beträgt.

8. Impfpistole nach einem der Ansprüche 5-7, **dadurch gekennzeichnet, dass** die Thermistoren (33)

- eine Größe von kleiner als 1 mm, insbesondere kleiner als 0,5 mm, bevorzugt kleiner als 0,1 mm aufweisen, und/oder
- in eine kugelförmige Glashülle mit einem Durchmesser von $\geq 1$ mm, $\geq 0,5$ mm, bevorzugt $\geq 0,1$ mm, eingekapselt sind, und/oder
- ein negativen Temperaturkoeffizienten aufweisen, insbesondere auf Siliziumbasis gefertigt sind, oder
- einen positiven Temperaturkoeffizienten aufweisen, insbesondere Platindraht umfassen.

9. Impfpistole nach einem der Ansprüche 4-8, **dadurch gekennzeichnet, dass** der Flüssigkeitssensor (3) ein hohlzylindrisches Rohr (30) umfasst, wobei insbesondere ein Außendurchmesser ($D_a$) des Rohres (30) $\leq 5$ mm, bevorzugt $\leq 1$ mm und ein Innendurchmesser ($D_i$) des Rohres $\leq 4,5$mm, bevorzugt $\leq 0,9$ mm ist, und wobei ein Innenraum des Rohres (30) mit einem Verfüllmaterial (32), insbesondere einem Epoxidharz, ausgegossen ist, wobei die Thermistoren (33) in einer Stirnseite des Füllmaterials (32) nebeneinander, bevorzugt in einem Abstand (a), der zwischen dem 0,5 und 0,8-fachen des Innendurchmessers ($D_i$) beträgt, angeordnet sind und bevorzugt über die Stirnseite hervorragen.

10. Impfpistole nach einem der Ansprüche 1-9, **dadurch gekennzeichnet, dass** die Steuerung (4)

- eine analoge Ansteuerschaltung (40), insbesondere

○ eine Brückenschaltung mittels welcher ein zu einer Temperaturdifferenz zwischen den Thermistoren (NTC1, NTC2) proportionales analoges Messsignal, oder
○ eine einen Spannungsteiler aus jeweils einem Thermistor (NTC1, NTC2) und einem Referenzwiderstand (R8, R10, R12) umfassende Schaltung (40), mittels welcher zwei zu der Temperatur der Thermistoren proportionale analoge Messsignale (CH1, CH2) erfassbar sind, und

- einen Mikrocontroller (41) und einen, insbesondere in den Mikrocontroller integrierten, Datenspeicher (42), zur

○ Speicherung und Ausführung eines Steuerungsalgorithmus zum Ansteuern des Flüssigkeitssensors (3), insbesondere mittels einer Ansteuerschaltung (40), und erfassen und, mittels eines Analog-Digitalwandlers des Mikrocontrollers (41), digitalisieren eines oder mehrerer analoger Messsignale und Ableiten eines digitalen Ausgabesignals aus den digitalisierten Messsignalen, wobei das digitale Ausgabesignal insbesondere eine Temperaturdifferenz zwischen den Thermistoren (NTC1, NTC2) oder die Temperaturdifferenz im Verhältnis zu einer Heizleistung ($P_H$) oder einer Messsleistung ($P_M$), mit welcher einer der Thermistoren (NTC2) beaufschlagt wird, wiedergibt, und/oder
○ Ausführen eines Auswertealgorithmus, welcher aus dem digitalisierten Messsignal das Vorhandensein oder Nichtvorhandensein eines Fehlerszustandes ableitet, und/oder

- einen Mikrocontroller

○ zur Freigabe des Ladevorgangs und/oder Ausstoßvorgangs, und/oder
○ Ausführen eines Auswertealgorithmus, welcher aus dem digitalisierten Messsignal das Vorhandensein oder Nichtvorhandensein eines Fehlerszustandes ableitet, umfasst.

11. Impfpistole nach Anspruch 10, **dadurch gekennzeichnet, dass** die Steuerung (4) dazu vorbereitet ist, bei der Heizung und/oder der Messung der Temperaturdifferenz den Messthermistor (33m) mit einem zeitlich konstanten Strom oder einer zeitlich konstanten Leistung zu beaufschlagen.

12. Impfpistole nach Anspruch 10, **dadurch gekennzeichnet, dass** die Steuerung (4) dazu vorbereitet ist, die Messung der Temperaturdifferenz periodisch zu wiederholen, wobei die Messung in drei Phasen unterteilt ist, nämlich

- eine Heizphase, welche für eine Heizdauer ($T_H$) andauert und in welcher die Steuerung den Messthermistor mit einem für die Dauer der Heizphase konstanten Strom ($I_H$) oder einer konstanten Leistung ($P_H$) beaufschlagt,
- eine Ausgleichsphase, welche für eine Ausgleichsdauer ($T_A$) andauert und in welcher der Messthermistor mit einem Strom oder einer Leistung von 0 beaufschlagt wird, und
- einer sich an die Ausgleichsphase anschließende Messphase von einer Messdauer ($T_M$), in der der Messthermistor mit einem konstanten Messstrom ($I_M$) und/oder einer konstanten Messleistung ($P_M$) beaufschlagt wird, wobei die Erfassung und Digitalisierung des oder der analogen Messsignale zu einem Messzeitpunkt erfolgt, welcher lediglich eine im Vergleich zur Messdauer ($T_M$) kurze Zeit beansprucht und insbesondere am Ende der Messphase liegt.

13. Impfpistole nach Anspruch 12, **dadurch gekennzeichnet, dass** die Heizdauer ($T_H$) weniger als 100 ms, insbesondere weniger als 70 ms, bevorzugt zwischen 50 und 70 ms und die Ausgleichsdauer ($T_A$) weniger als 50 ms, insbesondere zwischen 20 und 30 ms und eine Messperiode (T), also die Summe aus Heizdauer, Ausgleichsdauer und Messdauer, weniger als 200ms bevorzugt weniger als 100ms beträgt.

14. Impfpistole nach einem der Ansprüche 12 oder 13, **dadurch gekennzeichnet, dass** der Heizstrom ($I_H$) gleich dem Messstrom ($I_M$) oder die Heizleistung ($P_H$) gleich der Messleistung (PM) ist.

**15.** Impfpistole nach einem der Ansprüche 1-14 **dadurch gekennzeichnet, dass** die Steuerung (4) in unmittelbarer Nähe zum Flüssigkeitssensor (3) zwischen der Hochdruckkammer (2) und einem diese einschließenden Gehäuse (10) angeordnet ist.

**16.** Verfahren zur Ermittlung eines Fehlerzustandes einer Impfpistole gemäß einem der Ansprüche 1-15, **dadurch gekennzeichnet, dass**
während eines Ladevorgangs der Impfpistole umfassend eine Unterdruckerzeugungsphase und eine anschließende Flüssigkeitseinströmphase, mittels eines Flüssigkeitssensors (3) ein Signal erfasst wird, aus dem durch eine Steuerung (4) das Vorhandensein von Flüssigkeit in der Hochdruckkammer und eine Ist-Einströmrate von Impfflüssigkeit durch den Einlass (21) in die Hochdruckkammer (2) ableitbar ist, wobei

- sofern ein Vergleich der Ist-Einströmrate mit einer Soll-Einströmrate während der Einströmphase ergibt, dass die tatsächliche Flüssigkeitseinströmrate sehr viel weniger, insbesondere weniger als 20%, der Soll-Einströmrate beträgt, durch die Steuerung (4) abgeleitet wird, dass ein Fehlerzustand "Einströmventil defekt geschlossen ODER Reservoir vollständig leer" abgeleitet wird, und
- sofern die Ist-Einströmrate weniger aber nicht viel weniger, insbesondere zwischen 20% und 80% der Soll-Einströmrate während der Einströmphase beträgt, durch die Steuerung (4) das Vorliegen eines Fehlerzustandes "Ausströmventil defekt offen ODER Reservoir nahezu leer" abgeleitet wird, und

wobei insbesondere ein Auslösen der Impfpistole unterbunden wird, sofern ein Fehlerzustand vorliegt.

**17.** Verfahren zur Ermittlung eines Fehlerzustandes einer Impfpistole gemäß einem der Ansprüche 1-15, **dadurch gekennzeichnet, dass**
während eines Ausstoßvorgangs unter Erzeugung eines Hochdrucks in der Hochdruckkammer (2) mittels eines Flüssigkeitssensors (3) ein Signal erfasst wird, aus welchem durch eine Steuerung (4) eine Ist-Strömungsrate durch den Einlass (21) aus oder in die Hochdruckkammer und/oder das Vorhandensein von Flüssigkeit in der Hochdruckkammer ableitbar ist, und,
sofern die gemessene Ist-Strömungsrate größer als 0 ist, die Steuerung (4) das Vorliegen eines Fehlerzustandes "Einlassventil defekt offen" ableitet, und insbesondere einen anschließenden Ladevorgang und/oder Ausstoßvorgang unterbindet.

**18.** Verfahren gemäß Anspruch 16 und/oder 17, **dadurch gekennzeichnet, dass** die Steuerung (4) einen ermittelten Fehlerzustand einem Benutzer der Impfpistole mittels eines Anzeigemittels mitteilt.

**A**

**B**

**Fig. 1**

**A**

**B**

$$0,8 D_i \geq a \geq 0,5 D_i$$

**Fig. 2**

40

**Fig. 3A**

**Fig. 4**

**Fig. 3B**

**Fig. 5A**

**Fig. 5B**

EP 3 854 434 A1

Europäisches Patentamt
European Patent Office
Office européen des brevets

**EUROPÄISCHER RECHERCHENBERICHT**

Nummer der Anmeldung

EP 20 21 1511

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | EP 1 515 763 B1 (INTERVET INT BV [NL]) 17. August 2011 (2011-08-17) | 1-18 | INV. A61M5/30 |
| Y | * das ganze Dokument * | 1-18 | A61M5/20 |
| Y | WO 2018/203203 A1 (TARGET POINT TECH LTD [IL]) 8. November 2018 (2018-11-08) * Seite 13 - Seite 36; Abbildungen 1-19 * | 1-18 | ADD. A61D1/02 |
| A | DE 100 84 999 T1 (FLUID COMPONENTS INTL [US]) 19. September 2002 (2002-09-19) * das ganze Dokument * | 1-18 | |
| A | EP 2 514 458 A2 (MARK ANDERSON AND ASSOCIATES INC [US]) 24. Oktober 2012 (2012-10-24) * das ganze Dokument * | 1-18 | |

RECHERCHIERTE SACHGEBIETE (IPC)

A61M
A61D

Der vorliegende Recherchenbericht wurde für alle Patentansprüche erstellt

| Recherchenort | Abschlußdatum der Recherche | Prüfer |
|---|---|---|
| München | 16. Juni 2021 | Preller, D |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................

& : Mitglied der gleichen Patentfamilie, übereinstimmendes Dokument

EPO FORM 1503 03.82 (P04C03)

19

**ANHANG ZUM EUROPÄISCHEN RECHERCHENBERICHT**
**ÜBER DIE EUROPÄISCHE PATENTANMELDUNG NR.**

EP 20 21 1511

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-06-2021

| Im Recherchenbericht angeführtes Patentdokument | Datum der Veröffentlichung | Mitglied(er) der Patentfamilie | Datum der Veröffentlichung |
|---|---|---|---|
| EP 1515763 B1 | 17-08-2011 | AT 520434 T | 15-09-2011 |
| | | AU 2003245919 A1 | 22-12-2003 |
| | | CA 2486398 A1 | 18-12-2003 |
| | | DK 1515763 T3 | 21-11-2011 |
| | | EP 1515763 A1 | 23-03-2005 |
| | | ES 2368040 T3 | 11-11-2011 |
| | | JP 4499555 B2 | 07-07-2010 |
| | | JP 2005528958 A | 29-09-2005 |
| | | US 2006217661 A1 | 28-09-2006 |
| | | WO 03103751 A1 | 18-12-2003 |
| WO 2018203203 A1 | 08-11-2018 | BR 112019022395 A2 | 19-05-2020 |
| | | CN 110769872 A | 07-02-2020 |
| | | EP 3618896 A1 | 11-03-2020 |
| | | RU 2019136958 A | 19-05-2021 |
| | | US 2020061284 A1 | 27-02-2020 |
| | | WO 2018203203 A1 | 08-11-2018 |
| DE 10084999 T1 | 19-09-2002 | AU 7829600 A | 17-04-2001 |
| | | CA 2384788 A1 | 22-03-2001 |
| | | CN 1390297 A | 08-01-2003 |
| | | DE 10084999 T1 | 19-09-2002 |
| | | EP 1214565 A1 | 19-06-2002 |
| | | US 6208254 B1 | 27-03-2001 |
| | | US 2002130780 A1 | 19-09-2002 |
| | | WO 0120269 A1 | 22-03-2001 |
| EP 2514458 A2 | 24-10-2012 | AU 2005253993 A1 | 29-12-2005 |
| | | CA 2568355 A1 | 29-12-2005 |
| | | EP 1773424 A2 | 18-04-2007 |
| | | EP 2514458 A2 | 24-10-2012 |
| | | US 2008071218 A1 | 20-03-2008 |
| | | US 2011224613 A1 | 15-09-2011 |
| | | WO 2005122722 A2 | 29-12-2005 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1515763 B1